# EUROPEAN PATENT APPLICATION

(11) **EP 3 378 508 A1**
(43) Date of publication of application: **26.09.2018**
(21) Application number: 18162789.4
(22) Date of filing: 20.03.2018
(51) Int. Cl.: A61M 5/00

(54) **CONTAINER FOR CANNULAS**

(30) Priority: 21.03.2017 ES 201730306 U
(71) Applicant: FAES FARMA, S.A., 48940 Leioa - Vizcaya (ES)
(72) Inventor: HERNÁNDEZ HERRERO, Gonzalo, 48940 Leioa, Vizcaya (ES); TATO CERDEIRAS, Paloma, 48940 Leioa, Vizcaya (ES); SERRA BARCHÍN, Marc, 17813 La Vall de Bianya, Girona (ES); VILAR ROSANAS, Marc, 17813 La Vall de Bianya, Girona (ES); BARNEDA DARIAS, David, 17813 La Vall de Bianya, Girona (ES)
(74) Representative: ABG Patentes, S.L.

(57) **Abstract**

The present invention relates to a medical device for the application or delivery of drugs, and particularly to a container for storing and protecting cannulas or other objects with a substantially elongated shape for the purpose of preserving hygiene and suitability conditions for therapeutic use, the container comprising a plurality of substantially frustoconical cavities.

## Description

### Object of the Invention

The invention is comprised in the field of medical devices for the application or delivery of drugs and relates to a container for storing and protecting cannulas or other objects with a substantially elongated shape.

### Background of the Invention

A number of therapeutic treatments require the administration of a medicinal substance to the patient by means of devices configured for that purpose, such as cannulas, inhalers, or hypodermic needles. For the sake of fundamental hygiene, these devices must be stored protected from contaminants and disposed of once they are used.

The use of individual packages, such as sealed pouches or sachets, for storing these devices is common in the pharmaceutical industry. However, packages of this type are difficult for people without medical training to handle, and these people may inadvertently contaminate or incorrectly use the device.

Furthermore, the package must often be handled with both hands and be coupled at the same time to the recipient containing the medicinal product.

### Description of the Invention

The present invention proposes a solution to the aforementioned problems by means of a container according to claim 1. Preferred embodiments of the invention are defined in the dependent claims.

A first inventive aspect provides a *container adapted for containing a plurality of essentially frustoconical disposable cannulas, characterized in that it comprises*
*a base comprising,*
*a lower end and an upper end,*
*a support surface at the lower end thereof,*
*a plurality of substantially frustoconical cavities, with an inner wall and an outer wall, configured for tightly housing and retaining the plurality of disposable cannulas, and*
*a plurality of reinforcement walls arranged between the outer walls of at least two cavities, and*
*a cover configured for closing the base at the upper end thereof.*

Throughout this document, *cannula* will be understood as being an essentially prismatic, cylindrical, or frustoconical element that can be used for projecting or delivering a fluid substance to an area of application of the treatment.

*Container* will be understood as being a structural element capable of storing, containing, protecting, and arranging in a given position the cannulas, preferably made of an essentially rigid and lightweight material, and suitable for containing sanitary material.

*Cavity* will be understood as being a notch of the base the function of which is to house and hold or retain the cannulas, such that they are kept in a given position, making them easier to use.

Advantageously, the container allows retaining one or more cannulas in respective cavities by friction fit, such that when the container is opened by removing the cover, the cannulas are in a position making them easier to use. Also advantageously, the reinforcement walls are arranged such that they improve the rigidity of the container and allow constructing the assembly with a smaller amount of material.

In a particular embodiment, the support surface comprises support columns.

*Support column* will be understood as being the element of the container which allows raising the support surface with respect to the level of the surface on which the container rests, and which allows supporting the container in a stable manner in combination with other support columns.

In another particular embodiment, the base is substantially rectangular.

Advantageously, the rectangular base allows distributing the cannulas forming rows and/or columns.

In another particular embodiment, the support columns of the support surface are arranged in the corners.

Advantageously, the arrangement of the support columns at the end points of the base gives the container greater stability and allows making better use of the space available for storing cannulas.

In a particular embodiment, the cavities are configured for retaining the plurality of disposable cannulas by means of friction fit or press fit.

In another particular embodiment, the cannulas comprise a flange on its cross section configured for assuring the holding of the cannula in the cavity.

Advantageously, retention by means of friction fit or press fit allows holding the cannulas in place without additional elements, and at the same time it allows the cannulas to be removed for use by means of gently pulling on them.

In another particular embodiment, the cover comprises a gripping area for a secure holding thereof.

Advantageously, the cover can be removed by a user pulling on it while holding the gripping area with the fingers.

In yet another particular embodiment, the upper end of the base comprises a recess configured for receiving the cover, providing a substantially tight closure of the base and the cover of the container by means of friction fit or press fit.

Advantageously, the substantially tight closure of the container allows a precise and secure closure of the cover with respect to the base, and at the same time it allows opening by means of gently pulling on the cover.

In a particular embodiment, the reinforcement walls are arranged in an alternating manner in parallel and in perpendicular.

Advantageously, the alternating arrangement of the reinforcement walls in parallel and in perpendicular allows an increase in rigidity of the assembly with a minimal use of material.

In a last particular embodiment, the base comprises a plurality of column walls arranged between the outer walls of the cavities and the support columns.

Advantageously, the column walls limit deformation of the support columns and increase structural rigidity of the base.

All the features and/or steps of methods described in this specification (including the claims, description, and drawings) can be combined in any combination, with the exception of combinations of such mutually exclusive features.

### Description of the Drawings

These and other features and advantages of the invention will be more clearly understood based on the following detailed description of a preferred embodiment, given only by way of nonlimiting illustrative example in reference to the attached drawings.
Figure 1 shows a perspective view of the container with the cover being closed.
Figure 2a illustrates a perspective view of the container showing its lower part.
Figure 2b shows a bottom view of the container, in which the arrangement of the reinforcement walls can be seen.
Figure 3 shows a view of the container with the cover being lifted off, which allows seeing the internal arrangement of the cannulas.

### Detailed Description of the Invention

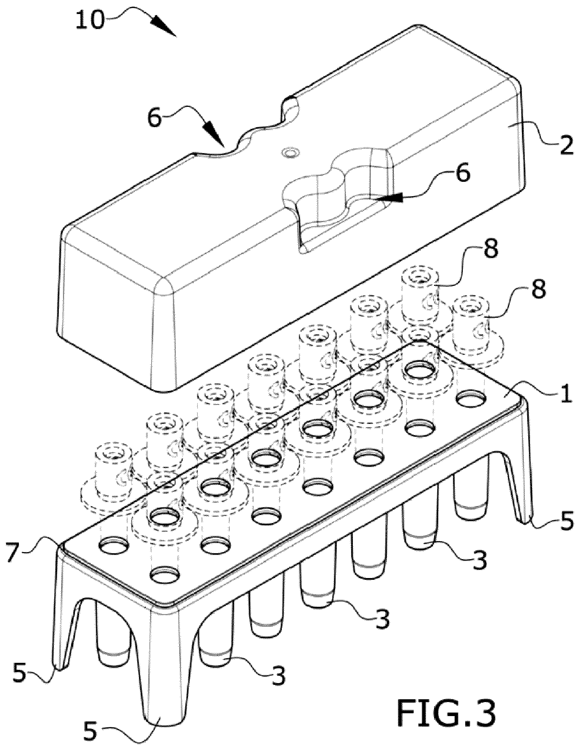

The container (10) that is described below is preferably adapted for containing disposable cannulas (8) for applying a fluid medicinal product. Said cannulas (8), shown using dashed lines in Figure 3, consist of substantially elongated, hollow frustoconical cylinders, the widest end of which comprises a portion having a variable section intended for being coupled with the dispensing element of a medicinal product package.

The container (10) has the function of storing, containing, protecting, and arranging in a given position the cannulas (8), fundamentally for the purpose of preserving hygiene and suitability conditions for therapeutic use, and for allowing the safe storage thereof until the moment of use. Another function of the container (10) is to arrange the cannulas (8) such that they can be used, i.e., coupled to the medicinal product dispenser, without having to touch the cannulas (8) directly with the hands. This therefore makes it easier for the product to be handled by people without specific training.

The container (10) may be produced, without prejudice to the use of other materials and other techniques, by means of an injection molding process using a plastic material, such as a polymer, for example. The container (10) is therefore lightweight, easy to produce, uses a minimal amount of material, and complies with the functions of the container (10) defined above.

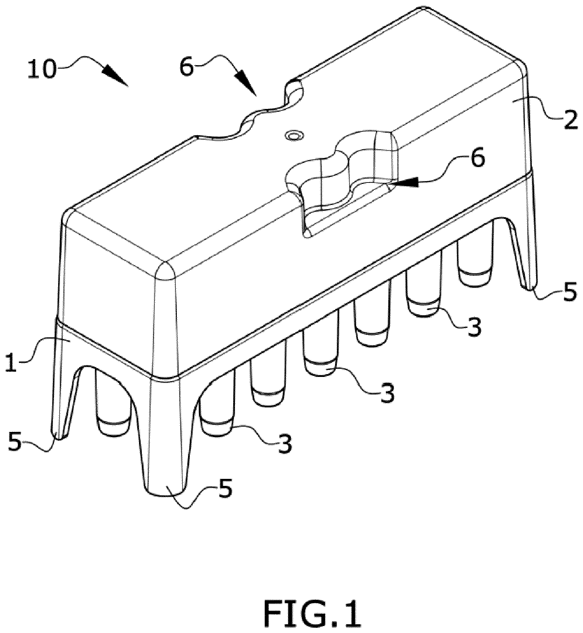

Figure 1 shows a particular embodiment of the container (10), having a rectangular base and a capacity for 14 cannulas (8) arranged individually in two rows of 7 units. In other non-depicted embodiments, each cavity (3) may contain more than one cannula (8), with the shape of said cavities (3) being adapted accordingly for housing and retaining said cannulas (8).

In this embodiment, the container (10) comprises two parts, a base (1) and a cover (2). The cover (2) is closed on the upper part of the base (1), such that when the container (10) is closed it forms a compact and manageable assembly in which the cannulas (8) are protected against impacts, preserved, and contaminant-free.

The base (1), which consists of an essentially planar surface with support columns (5) in the corners, can be seen in greater detail in Figure 3. These four support columns (5) have a dual function: on one hand, they provide a stable platform for the base (1), and on the other they allow raising the assembly such that the receptacles of the cannulas (8) are located above the level of the support surface. The base (1) further comprises a recess (7) configured for receiving the cover (2) and providing a tight closure of the container (10).

The support columns (5) are conceived for providing the base (1) with a stable surface. In a particular embodiment not shown in the drawings, instead of a plurality of separate support columns (5), the same objective is achieved with a continuous element, or skirting, that goes around the entire base (1).

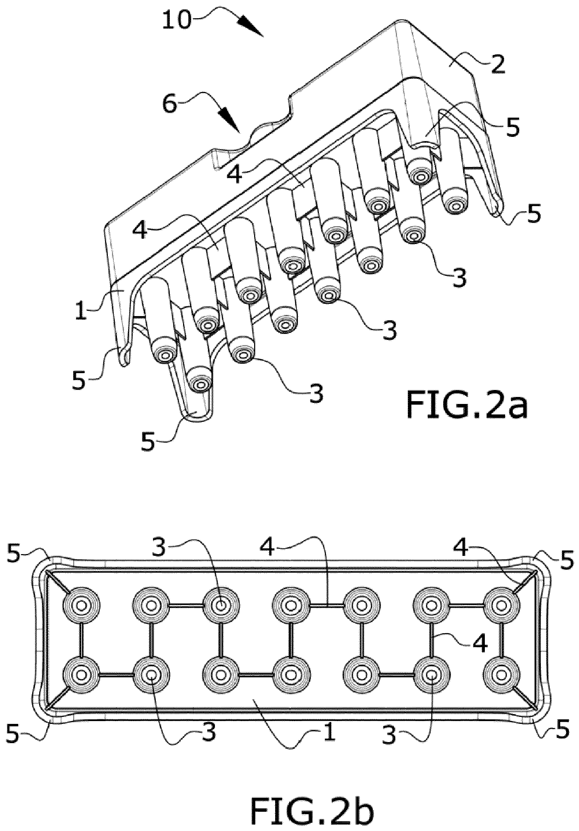

The cavities (3) have the function of containing the cannulas (8) and have a shape that is essentially identical to the shape of the cannulas (8), i.e., a frustoconical cylinder, but closed at the lower end thereof. As can be seen in Figure 2, the cavities (3) are arranged vertically according to their longitudinal axis, with their wider end being open in the upper portion of the base (1), and with their narrower end being closed for protecting the cannulas (8) and holding them. In the present embodiment, the cannulas (8) are held by means of press fit or interference fit, either at their upper end or lower end, or at both ends.

An additional feature of the cavities (3) which can be seen in Figure 1 is that their length is slightly less than the length of the support columns (5), such that when the container (10) is supported, the lower end of the cavities (3) does not come into contact with the support surface in order to prevent any possible contamination. Furthermore, this feature confers a certain degree of flexibility to the container (10), such that if the container (10) is loaded in excess in the upper part thereof and the support columns (5) slightly bend, the lower ends of the cavities (3) come into contact with the support surface, providing greater stability and preventing excessive deformation of the container (10).

Any number of cannulas (8), from one unit, can fit in the container (10), and the maximum number of cavities (3) is limited only by the dimensions of the container (10). As indicated, the embodiment shown in Figure 3 shows a container (10) with a capacity for 14 cannulas (8). The arrangement of the cannulas (8) in the vertical position offers the advantages indicated above, but it is also possible to arrange them in the horizontal or oblique position, at the expense of a less efficient use of space.

The cover (2) is configured as a hollow rectangular prism in which one of the bases has been removed. In the particular embodiment shown in Figure 1, the cover (2) comprises gripping areas (6) on its two longer sides, substantially in its central part, constituting a gripping area (6) so that a user may comfortably grip the cover (2) and separate it from the base (1). Optionally, said gripping areas (6) can be found in a number and in a position other than what is shown in Figure 1, such as in the corners of the cover (2), for example. An additional function of the cover (2) is to allow stacking containers (10) to make storage easier.

There are a series of reinforcement walls (4) arranged between the outer faces of the cavities (3), as can be seen in Figures 2a and 2b. The function of these reinforcement walls (4) is to serve as a structural reinforcement for the base (1) between the cavities (3). Complementary to the reinforcement walls (4), the base (1) further comprises column walls, which join the cavities (3) with the support columns (5), similar to the reinforcement walls (4), and the function of which is both structural and also to limit deformation of said support columns (5). Figure 2b shows in detail the particular arrangement of the reinforcement walls (4) and of the column walls: each reinforcement wall (4) joins two contiguous cavities (3), reinforcement walls (4) that are parallel and transverse to the longitudinal axis of the container (10) being arranged in an alternating manner, whereas the column walls join the cavities (3) of the corners with the support columns (5). This particular way of constructing the structural reinforcement elements (4) corresponds to a configuration of maximum structural rigidity and minimal use of material, without negatively affecting the injection process.

## Claims

1. A container (10) adapted for containing a plurality of essentially frustoconical disposable cannulas (8), **characterized in that** it comprises,
a base (1) comprising,
a lower end and an upper end,
a support surface at the lower end thereof,
a plurality of substantially frustoconical cavities (3), with an inner wall and an outer wall, configured for tightly housing and retaining the plurality of disposable cannulas (8), and
a plurality of reinforcement walls (4) arranged between the outer walls of at least two cavities (3), and
a cover (2) configured for closing the base (1) at the upper end thereof.

2. The container (10) according to claim 1, **characterized in that** the support surface comprises support columns (5).

3. The container (10) according to claim 1, **characterized in that** the base (1) is substantially rectangular.

4. The container (10) according to claim 3, **characterized in that** the support columns (5) of the support surface are arranged in the corners.

5. The container (10) according to any of the preceding claims, **characterized in that** the cavities (3) are configured for retaining the plurality of disposable cannulas (8) by means of friction fit or press fit.

6. The container (10) according to any of the preceding claims, **characterized in that** the cover (2) comprises a gripping area (6) for a secure holding thereof.

7. The container (10) according to any of the preceding claims, **characterized in that** the upper end of the base (1) comprises a recess (7) configured for receiving the cover (2), providing a substantially tight closure of the base (1) and the cover (2) of the container (10) by means of friction fit or press fit.

8. The container (10) according to any of the preceding claims, **characterized in that** the reinforcement walls (4) are arranged in an alternating manner in parallel and in perpendicular.

9. The container (10) according to any of the preceding claims, **characterized in that** the base (1) comprises a plurality of column walls arranged between the outer walls of the cavities (3) and the support columns (5).
